# EUROPÄISCHE PATENTANMELDUNG

(11) **EP 3 865 213 A2**
(43) Veröffentlichungstag der Anmeldung: **18.08.2021**
(21) Anmeldenummer: 21157071.8
(22) Anmeldetag: 15.02.2021
(51) Int. Cl.: B01L 3/00, C12Q 1/6841

(54) **VERFAHREN ZUM NACHWEIS VON LEBENDEN MIKROORGANISMEN UND EIN FLUIDISCHES KANALSYSTEM**

(30) Priorität: 14.02.2020 DE 102020103971; 12.02.2021 US 202117174398
(71) Anmelder: Testo bioAnalytics GmbH, 79822 Titisee-Neustadt (DE)
(72) Erfinder: VASILEUSKAYA-SCHULZ, Zinaida, 79111 Freiburg (DE); QUEHL, Paul, 37085 Göttingen (DE); BOOS, Stefanie, 78052 Villingen-Schwenningen (DE); RIEMER, Joel, 79874 Breitnau (DE); RUDEN, Serge, 79822 Titisee-Neustadt (DE); BRAGA, Daniel, 79115 Freiburg (DE)
(74) Vertreter: Mertzlufft-Paufler, Cornelius

(57) **Zusammenfassung**

Es wird vorgeschlagen, ein Verfahren (7) zum Nachweis von lebenden Mikroorganismen (1) und/oder zur Differenzierung von lebenden (12) und toten (13) Mikroorganismen in einer Probe (9) mittels in-situ-Hybridisierung (14), insbesondere einer Fluoreszenz-in-situ-Hybridisierung, und einer optischen Analyse (26) der nachzuweisenden Mikroorganismen (1) bereitzustellen, wobei der Probe (9) vor der Hybridisierung (14) wenigstens eine Substanz (10) beigegeben wird, die eine unterschiedliche Veränderung der RNA-Konzentration in toten Zellen und in lebenden Zellen begünstigt (Fig.3).

## Beschreibung

Die Erfindung betrifft ein Verfahren zum Nachweis von lebenden Mikroorganismen und/oder zur Differenzierung von lebenden und toten Mikroorganismen in einer Probe mittels in-situ-Hybridisierung, insbesondere einer Fluoreszenz-in-situ-Hybridisierung, und einer optischen Analyse der nachzuweisenden Mikroorganismen, wobei der Probe vor der Hybridisierung wenigstens eine Substanz beigegeben wird, die eine unterschiedliche Veränderung der Nukleinsäure-Konzentration, insbesondere RNA-Konzentration und/oder DNA-Konzentration, in toten Zellen und in lebenden Zellen begünstigt.

Zum Nachweis von Nukleinsäuren in einzelnen Zellen sind beispielsweise Verfahren wie In-Situ-Hybridisierung (ISH) sowie die Fluoreszenz-In-Situ-Hybridisierung (FISH) bekannt. Dabei werden kurze synthetische Nukleinsäuresonden eingesetzt, welche über Basenpaarungen an die nachzuweisende Zielsequenz binden. Die In-Situ-Hybridisierung und die Fluoreszenz-In-Situ-Hybridisierung, bei welcher die Nukleinsäuresonden fluoreszent markiert sind, können zur spezifischen Detektion von Nukleinsäuren (DNA und/oder RNA Molekülen) verwendet werden.

Die spezifische Detektion von Nukleinsäuren kommt beispielsweise bei einer Produktions- und/oder Qualitätskontrolle zur Anwendung. Für eine Vielzahl von Substanzen, Rohstoffen und Produkten aus den unterschiedlichen Bereichen der Industrie, Gesundheit oder Gastronomie ist es wichtig, dass die mikrobiologische Sicherheit gewährleistet werden kann. Beispielsweise kann bei einem Reinigungsverfahren einer Oberfläche auf diese Weise geprüft werden, ob die Reinigung/Desinfektion erfolgreich war.

Mit molekularbiologischen Verfahren, beispielsweise PCR, kann die Nukleinsäure eines Mikroorganismus in wenigen Stunden nachgewiesen werden. Dabei wird jedoch die gesamte DNA/RNA aller Keime von lebenden und toten Mikroorganismen ermittelt. Für hygienische Bewertungen sind jedoch nur die Mikroorganismen von Belang, die eine Infektion auslösen können, und nicht bereits abgestorbenen Mikroorganismen, die nicht mehr zu einer Erkrankung führen können. Daher ist es für ein Nachweisverfahren wichtig, dass eine Differenzierung zwischen lebenden und toten Mikroorganismen stattfindet.

Vor diesem Hintergrund ist es Aufgabe der vorliegenden Erfindung, ein einfaches und schnelles Verfahren zum Nachweis von Mikroorganismen in einer Probe bereitzustellen, das eine Unterscheidung lebender Mikroorganismen von toten Mikroorganismen ermöglicht und außerdem außerhalb von Laborumgebungen durchführbar ist.

Erfindungsgemäß wird diese Aufgabe durch die Merkmale von Anspruch 1 gelöst. Insbesondere wird somit erfindungsgemäß zur Lösung der genannten Aufgabe bei einem Verfahren der eingangs beschriebenen Art vorgeschlagen, dass der zu untersuchenden Probe vor der Hybridisierung wenigstens eine Substanz beigegeben wird, die eine unterschiedliche Veränderung der Nukleinsäure-Konzentration, insbesondere RNA-Konzentration und/oder DNA-Konzentration, in toten Zellen und in lebenden Zellen begünstigt.

Hierzu macht sich die Erfindung zunutze, dass eine Differenzierung zwischen lebenden und toten Mikroorganismen ermöglicht werden kann, indem der Probe vor der Hybridisierung wenigstens eine Substanz beigegeben wird, die eine unterschiedliche Veränderung der Nukleinsäure-Konzentration in toten Zellen und in lebenden Zellen begünstigt. Beispielsweise kann die RNA-Konzentration und/oder DNA-Konzentration in toten Zellen vermindert und/oder in lebenden Zellen erhöht werden. Alternativ oder zusätzlich kann die RNA-Konzentration und/oder DNA-Konzentration in toten und lebenden Zellen unterschiedlich schnell vermindert werden. Alternativ oder zusätzlich kann die Zugänglichkeit für Nachweisreagenzien zu RNA- und/oder DNA in toten oder lebenden Zellen modifiziert werden. Alternativ oder zusätzlich können weitere Nachweisreagenzien als Lebend- oder "Tot"-Indikatoren in die Zellen eingebracht werden und ebenfalls als Informationen ausgelesen werden.

Eine vorteilhafte Ausführungsform gemäß der Erfindung sieht vor, dass die beigegebene Substanz wenigstens einen chemischen Stoff enthält, der RNA und/oder DNA abbaut. Der chemische Stoff, der RNA und/oder DNA abbaut, kann ein Enzym sein. Beispielsweise ist das eine DNAse. Die beigegebene Substanz kann auch eine RNAse sein. Von Vorteil ist es dabei, dass eine RNAse in die toten Zellen, die viel permeabler sind als die lebenden Zellen, d.h. sich oft durch eine erhöhte Durchlässigkeit auszeichnen, eindringen und im Zellraum enzymatisch aktiv werden kann. Dadurch können tote Partikel entfernt werden, was eine Lebend/Tod-Differenzierung erheblich verbessern kann. Die beigegebene Substanz kann auch einen chemischen Stoff enthalten, der einen RNA-Abbau und/oder einen DNA-Abbau begünstigt. Beispielsweise ist dieser Stoff Ethylendiamintetraacetat (EDTA), der auch als Schutzstoff vor Metalloenzymen verwendet werden kann. In einer vorteilhaften Ausführungsform beträgt die Konzentration von EDTA 0,1-2 M, bevorzugt 0,1-1 M, besonders bevorzugt 0,5 M. Es können auch weitere Stoffe wie beispielsweise Diethylentriaminpentaessigsäure (DTPA), Triethylentetraminhexaessigsäure (TTHA), RNA-Helikase, Polymerase, Chaperon oder siRNA verwendet werden. Die beigegebene Substanz kann aber auch alternativ oder zusätzlich einen chemischen Stoff enthalten, der die Durchlässigkeit bei toten Zellen erhöht. Insbesondere ist es Murein-Hydrolase, Lysozym, Mutanolysin, Glukosaminidase, Peptidase oder Amidase. Als Peptidasen, d.h. Enzymen, die Proteine oder Peptide spalten, können Exopeptidasen und/oder Endopeptidasen verwendet werden, in Abhängigkeit von der Lokalisierung innerhalb der zu spaltenden Polypeptidkette. Alternativ oder zusätzlich kann in der beigegebenen Substanz auch ein selektives oder nicht selektives Nährmedium enthalten sein, das einen RNA-Aufbau und/oder DNA-Aufbau in lebenden Zellen begünstigt. Beispielsweise ist das ein Peptonwasser, CASO-Bouillon, DEV Lactose-Bouillon, MRS Medium, Thioglycolat Bouillon, Hirn-Herz-Infusion-Bouillon, Caseinpepton-Sojamehlpepton-Bouillon, GN-(gramnegativ) Anreicherungsbouillon n. Hajna und/oder LB-Medium.

Eine vorteilhafte Ausführungsform gemäß der Erfindung sieht weiterhin vor, dass die Mikroorganismen zumindest mit Beigabe der Substanzen inkubiert werden. Insbesondere erfolgt die Inkubation der Mikroorganismen vor der Hybridisierung. Die Inkubationszeit kann dabei so bemessen werden, dass noch keine Zellteilung stattfindet. Dadurch kann die Wahrscheinlichkeit, dass tote Zellen als falsch-positives Signal erfasst werden, um mehrere Größenordnungen gesenkt werden, da diese tote Partikel durch die Inkubation der Mikroorganismen vor der Hybridisierung stark reduziert werden.

Eine vorteilhafte Ausführungsform gemäß der Erfindung sieht vor, dass die Hybridisierung mindestens zwei der Schritte einer Fixierung, einer Permeabilisierung und einer Denaturierung umfasst. Insbesondere kann vorgesehen sein, dass die Fixierung durch eine Denaturierungssubstanz erfolgt. Dies ermöglicht eine vorteilhafte Zusammensetzung des Hybridisierungspuffers, bei dem dasselbe Mittel zur Fixierung und Denaturierung verwendet werden kann.

Es kann auch vorgesehen sein, dass in der Hybridisierung alle der Schritte einer Fixierung, einer Permeabilisierung und einer Denaturierung umfasst sind. Bevorzugt findet die Hybridisierung in einem Hybridisierungspuffer statt. Der Hybridisierungspuffer enthält als Denaturierungssubstanz beispielsweise eine ungiftige Substanz, insbesondere Guanidiniumchlorid und/oder Harnstoff. Als Denaturierungssubstanzen können alternativ oder zusätzlich auch Guanidiniumthiocyanat und/oder Formamid verwendet werden. Die Verwendung von Harnstoff ist jedoch bevorzugt.

Hierdurch wird sichergestellt, dass der Einsatz von Guanidiniumchlorid und/oder Harnstoff anstelle von wassergefährdeten Stoffen oder anstelle vom toxischen Formamid die Anwendung des erfindungsgemäßen Verfahrens ohne Labor ermöglicht. Von Vorteil ist dabei, dass die Reagenzien in trockenform vorgelagert und anschließend im Hausmüll entsorgt werden können. In einer vorteilhaften Ausführungsform beträgt die Konzentration des Guanidiniumchlorid 0,1-2 M, bevorzugt 0,1-1 M, besonders bevorzugt 0,5 M. Vorteilhaft ist dabei, dass Guanidiniumchlorid in einer Konzentration von über 0,25 M einen mit der Erhöhung der Konzentration zunehmenden Schutz vor RNAsen bieten kann. Alternativ kann eine Mischung aus Guanidiniumchlorid und Harnstoff in einer bevorzugten Konzentration von 5,3 M Harnstoff mit 0,5 M Guanidiniumchlorid verwendet werden. Dies ermöglicht einen verbesserten Schutz gegenüber RNAsen. Für ListeriaFISH (FISH mit Bezug auf Listeria) wird (muss) kein Denaturierungsagenz benötigt (werden).

Eine vorteilhafte Ausführungsform gemäß der Erfindung sieht vor, dass der Probe ein Detergens zugegeben wird, bevor die Hybridisierung, insbesondere ein Fixierungsschritt, abgeschlossen ist. Von Vorteil ist es dabei, dass ein effektives Eindringen von Nachweissonden in die Zelle ermöglicht werden kann, indem der Probe ein Detergens zugegeben wird. Beispielsweise ist das Detergens Natriumdodecylsulfat (SDS) . Für ListeriaFISH wird (muss) kein Denaturierungsagenz benötigt (werden).
In einer vorteilhaften Ausführungsform beträgt die Konzentration des SDS 0,003%-0,05%. Für ListeriaFISH beträgt (kann) die Konzentration von SDS 0,075% (betragen).Durch die Zugabe des Detergens bevor der Fixierungsschritt abgeschlossen ist, kann erreichbar sein, dass anschließend nicht-lysierte, individuelle Mikroorganismen nachgewiesen werden können. Insbesondere wird das Detergens nicht vor Beginn der Fixierung zugegeben. Die Zugabe des Detergens vor Beginn der Fixierung, bei der eine weitere Veränderung des Aufbaus der Mikroorganismen verhindert wird, würde den Nachweis der in den einzelnen Mikroorganismen durch die Hybridisierung erzeugten Reaktionsprodukte erschweren.

Alternativ oder zusätzlich kann vorgesehen sein, dass vor der Hybridisierung eine Lebend/Tot-Differenzierung der in der Probe enthaltenden Mikroorganismen erfolgt. Somit kann der Nachweis von ausschließlich lebenden Mikroorganismen ermöglicht werden.

Eine vorteilhafte Ausführungsform gemäß der Erfindung sieht vor, dass der Hybridisierungspuffer mindestens ein Denaturierungsmittel und eine RNAse-inhibierende Substanz enthält. Dadurch können die Nukleinsäurehybride stabilisiert und auch Schutz vor RNAsen gewährleistet werden. Für Lebend/tot-Differenzierung in ListeriaFISH werden (müssen) weder Denaturierungsmittel noch RNAse-Inhibitoren verwendet (werden).

Eine vorteilhafte Ausführungsform gemäß der Erfindung sieht vor, dass die RNAse-inhibierende Substanz aus Guanidiniumchlorid, Guanidiniumthiocyanat, Formamid, Dithiothreitol (DTT), Diethylpyrocarbonat (DEPC), Oligovinylsulfonsäure, Polyvinylsulfonsäure, Ethansulfonsäure, Heparin, Gluthation, natürlichen Nukleosiden und Nukleotiden ausgewählt ist. Für Lebend/tot-Differenzierung in ListeriaFISH werden (müssen) weder Denaturierungsmittel noch RNAse-Inhibitoren verwendet (werden).

Eine vorteilhafte Ausführungsform gemäß der Erfindung sieht vor, dass der Hybridisierungspuffer mindestens ein Salz, vorzugsweise Natriumchlorid enthält. Durch die Verwendung von Salzen im Hybridisierungspuffer kann die Renaturierungsrate der doppelsträngigen Nukleinsäurehybriden und somit auch die Hybridisierungseffizienz erhöht werden. Zusätzlich können auch andere Salze wie beispielsweise Magnesiumchlorid und/oder Kaliumchlorid enthalten sein. Der Zusatz von Magnesiumchlorid kann eine Förderung der Hybridbildung ermöglichen. In einer vorteilhaften Ausführungsform beträgt die Konzentration von Natriumchlorid 25-1100 mM, bevorzugt 750-1000 mM, besonders bevorzugt 800-900 mM. Für ListeriaFISH wird (kann) Natriumchlorid in Konzentration von 1250mM verwendet (werden). Die bevorzugte Konzentration von Magnesiumchlorid beträgt 0,01-50 mM.

Es kann vorgesehen sein, dass der Hybridisierungspuffer als Puffersubstanz Tris(hydroxymethyl)aminomethanhydrochlorid (TRIS-HCl) enthält. Vorteilhaft ist dabei, dass die Puffersubstanz den pH-Wert des Puffers zwischen 5,5 und 8,7 stabilisiert. In einer vorteilhaften Ausführungsform beträgt die Konzentration der Puffersubstanz 10-100 mM.

Zusätzlich können RNA-Oligonukleotide als "Opfer-Substrat" in dem Hybridisierungspuffer enthalten sein. Dadurch ist ein verbesserter initialer Schutz vor RNAse Aktivität erreichbar. In einer vorteilhaften Ausführungsform beträgt die Konzentration von RNA-Oligonukleotiden 0,1-100 µM.

Eine vorteilhafte Ausführungsform gemäß der Erfindung sieht vor, dass die optische Analyse einen Schritt der Detektion, vorzugsweise des Quantifizierens der Mikroorganismen mit hybridisierten Nukleinsäuresonden umfasst. Alternativ oder zusätzlich kann die optische Analyse mittels Einzeldetektion der Mikroorganismen erfolgen. Somit kann eine absolute Quantifizierung der nachzuweisenden Organismen auf Basis einer Partikelmessung ermöglicht werden.

Eine vorteilhafte Ausführungsform gemäß der Erfindung sieht vor, dass die Nukleinsäuresonde komplementär zu einer RNA eines nachzuweisenden Mikroorganismus ist. Vorzugsweise kann die Nukleinsäuresonde aus linearen Oligonukleotidsonden ausgewählt werden. Beispielsweise sind das monoProbes, dopeProbes, tetraProbes und multiProbes. Die Nukleinsäuresonde kann auch aus Nukleinsäuresonden mit Sekundärstruktur ausgewählt werden. Beispielsweise sind das Molecular Beacons und Scorpions Sonden. Dadurch sind eine höhere Fluoreszenzintensität sowie ein besseres Signal-RauschVerhältnis erreichbar, was insbesondere für eine automatisierte Anwendung vorteilhaft ist.

Eine vorteilhafte Ausführungsform gemäß der Erfindung sieht vor, dass die Nukleinsäuresonde mit einem detektierbaren Marker verbunden ist. Der detektierbare Marker kann beispielsweise ein Fluoreszenzmarker, ein Chemolumineszenzmarker, ein Affinitätsmarker oder eine enzymatisch aktive Gruppe sein. Somit ist eine optische Detektion erreichbar. Der Affinitätsmarker kann beispielsweise Affinitätsbindungspaare Biotin-Streptavidin oder Antigen-Antikörper umfassen. Der enzymatische Marker kann beispielsweise Peroxidase, vorzugsweise Meerrettich-Peroxidase, oder Phosphatase, vorzugsweise alkalischer Phosphatase, sein.

Besonders vorteilhaft ist es, wenn eventuell entstehende Hintergrundfluoreszenz bzw. unspezifische Fluoreszenz in den hier beschriebenen FISH-Verfahren reduziert oder eliminiert wird. Dadurch können insbesondere automatisierte Detektionsverfahren spezifischer oder mit einem besseren Detektionslimit agieren. Eine unspezifische Fluoreszenz kann durch verschiedene Umstände bewirkt werden. Dazu gehören auch:
- Die unvollständige Quenching-Effizienz der eingesetzten Quencher-Moleküle. Insbesondere bei hohen Konzentrationen von eingesetzten FISH-Sonden entsteht hierdurch eine hohe unspezifische Hintergrundfluoreszenz durch überschüssige (nicht an die Ziel-RNA/DNA gebundenen) FISH-Sonden.
- Die FISH-Sonden können unspezifisch an nicht Target-Sequenzen binden oder sind unvollständig geschlossen und daher nicht oder unvollständig gequencht.

Als Gegenmaßnahmen zu der o.g. Hintergrundfluoreszenz und zur signifikanten Verbesserung des Signal-Rauschverhältnisses können daher folgende Methoden angewandt werden (eine oder einer Kombination aus diesen):
- Fluoreszenzkaskade: Gemäß/analog zum Försterresonanzenergietransfer wird ein zweiter Farbstoff in das Verfahren integriert. Die Probe wird mit einer Lichtquelle einer Wellenlänge bestrahlt, durch welche der Farbstoff/Reporter der FISH-Sonden nicht fluoresziert und somit keine Hintergrundfluoreszenz erzeugt. Stattdessen wird ein zweiter Farbstoff (hier: "Fluoreszenzdonor" mit Donorfluorophor) in den Assay eingebracht, welcher durch die eingebrachte Anregungswellenlänge fluoresziert und das emittierte Licht (durch die Fluoreszenzverschiebung in einen längerwelligen Bereich) wiederum in der Lage ist, den Farbstoff der z.B. FISH-Sonden / Molecular Beacons (hier: "Fluoreszenzakzeptor" mit Akzeptorfluorophor) zur Fluoreszenz anzuregen. Das Emissionsspektrum in welchem das Donorfluorophor fluoresziert, liegt dabei außerhalb oder nur in geringen Teilen innerhalb des Detektionsspektrums / der Detektionswellenlängen des Detektors und erzeugt daher keine relevante Hintergrundfluoreszenz. Der Mechanismus dieser zweistufigen Fluoreszenz funktioniert nur bzw. nur ausreichend, wenn Donor- und Akzeptorfluorophor in ausreichender räumlicher Nähe sind und nicht durch ein Quenchermolekül gequencht sind. Eingesetzte FISH-Sonden können bei diesem Verfahren die Fluoreszenzakzeptor oder die Fluoreszenzdonor-Funktion oder auch beide Funktionen übernehmen. Ideal geeignet sind hierbei Kombinationen aus jeweils mit dem Donor- und Akzeptorfluorophor versehene Oligos (DNA/RNA-Moleküle), welche in unmittelbare Nähe zueinander gebracht werden. Hierzu werden aneinandergrenzende oder durch Sekundärstrukturen nah beieinander liegende Bindestellen innerhalb des Zielorganismus gewählt, an welchen die Oligos (Fluoreszenzdonor und -Akzeptor) binden. Das Akzeptor- oder Donorfluorophor kann jedoch auch ein z.B. profluoreszenter Farbstoff sein und durch Enzyme innerhalb der Zielzellen zu einem für den Nachweis nutzbaren Fluorophor umgesetzt werden (z.B. Carboxyfluorescein) oder sich in bestimmten Zellstukturen (wie RNA, DNA, Proteinen und Lipiden) anlagern (Farbstoffe wie z.B. SYBR green, Ethidiumbromid, Coomassie) oder sich innerhalb des Zielorganismus akkumulieren (z.B. Tetramethylrhodamin-methylester) und somit ebenfalls in ausreichende räumliche Nähe des Donor- bzw. Akzeptorfluorophors gebracht werden. Besonders vorteilhaft können dabei Verfahren sein, welche entweder den Donor- oder den Akzeptorfluorophor mit einem unspezifischen Target (das Target kann z.B. RNA- oder DNA sein) in ausreichend Nähe des jeweils anderen bringen, da so Kosten für die spezifische Synthese von z.B. spezifischen FISH-Sonden vermieden werden können. Gleichzeitig lässt sich das Verfahren so standardisiert für die Verbesserung von anderen FISH-Assays und unabhängig von deren spezifischen Zielsequenzen verwenden. Insbesondere der Einsatz von Fluorophorgelabelten "random" Oligos, wie z.B. "random Hexamers", oder anderen zufälligen Oligo-Sequenzen ist dabei vorteilhaft, da eine Mischung aller möglichen (z.B.) Oligo-Hexamer-Sequenzoptionen (dies ist die Bedeutung von "random Hexamers") an allen einzelsträngigen Sequenzoptionen von Nukleinsäuren binden können. Diese "random" Oligos können sowohl am 3' und/oder am 5'-Ende mit einem oder mehreren Farbstoffen gelabelt sein. Durch die Nutzung einer Kombination von Fluoreszenz-Donoren- und -Akzeptoren ist es außerdem möglich, generell auf den Einsatz von Quenchern beim hier beschriebenen FISH-Verfahren zu verzichten und so kostengünstigere FISH-Sonden (ohne Quenchermoleküle) und bei gleicher Spezifität zu erstellen, da die Spezifität durch die notwendige räumliche Nähe der gebundener Farbstoffe (z.B. zwei spezifische Oligos mit Farbstoffen) erreicht werden kann. Für die Nutzung von unspezifischen Fluoreszenzdonoren oder -Akzeptoren ist darauf zu achten, dass diese erst nach dem Annealingschritt ("Bindungsschritt") mit dem spezifischen Reagenz (spezifische FISH-Sonde) eingebracht werden, da diese sonst ggf. die Bindungspositionen der spezifischen FISH-Sonden besetzen und zu falsch-negativen Ergebnissen führen. Werden profluoreszierende Farbstoffe eingesetzt, welche zunächst z.B. durch Enzyme der angefärbten Zellen umgesetzt werden müssen oder sich nur in Zellen mit einer ausreichend intakten Zellemembran akkumulieren, so lassen sich daraus zusätzlich Schlussfolgerung hinsichtlich der Vitalität der zu markierenden Zielorganismen treffen.
- Quenching-Sonden nach Annealing-Schritt: Falsch-gebundene oder unzureichend geschlossene FISH-Sonden ("molecular beacons") können erneut oder mit besserer Effizienz gequencht werden. Hierzu werden nach dem Annealingschritt der FISH-Sonden weitere Oligos eingebracht, welche komplementär zu den eingesetzten FISH-Sonden sind. Diese Oligos (hier: "Quencher-Oligos") tragen ein oder mehrere Quenchermoleküle an deren Enden und binden an den FISH-Sonden. Die komplementäre Sequenz ist dabei länger als die hairpin-bildende Halssequenz der FISH-Sonden und führen daher nach deren Annealing ("Bindung") an den FISH-Sonden zu einer stabilen linearisierten (zweisträngigen) Struktur. In diesem Struktur liegen die Fluorophore der FISH-Sonden nun durch den Quencher der "Quencher Oligos" gequencht vor und erzeugen nur noch eine geringe Hintergrundfluoreszenz.
- Immobilisierung von freien FISH-Sonden vor Messung: Überschüssige FISH-Sonden können aus dem Probenansatz entfernt werden. Hierzu wird (kann) das Probengemenge, vor der Messung und nach dem Annealingschritt der FISH-Sonden an die nachzuweisenden Zielmoleküle, über eine Oberfläche geführt (werden), welche die überschüssigen (nicht an Zielsequenzen gebundene FISH-Sonden) bindet und vor einer Messung der Probe aus dem Ansatz entfernt. Alternativ können auch Körper (wie z.B. "beads" / Kügelchen) zu der Probe hinzugegeben werden, an welchen die überschüssigen FISH-Sonden binden. Anschließend werden (können) diese Körper mitsamt der überschüssigen FISH-Sonden aus dem Reaktionsansatz abgeschieden (werden). Die Oberflächen bzw. Körper sind (können) dabei in beiden Fällen funktionalisiert (sein). Entweder werden sie dabei mit zu den FISH-Sonden komplementären Oligos (z.B. DNA- oder RNA-Fragmente) oder mit anderen Hilfsmitteln (z.B. gegen die Fluorophore oder Quencher der FISH-Sonden gerichteten Antikörpern) beschichtet/funktionalisiert. Typischerweise können hierzu biotinylierte komplementäre Oligos eingesetzt werden, welche an eine Strepatividin-beschichte Oberfläche gebunden sind. Ebenfalls ist es möglich die eingesetzten FISH-Sonden schon von Beginn an mit möglichen Bindungshilftsmittel (wie z.B. Biotin oder Streptavidin) zu koppeln und die zur Immobilisierung eingesetzten Oberflächen/Körper mit einem komplementären Bindungsmittel zu funktionalisieren (z.B. FISH-Sonden mit Biotin gekoppelt und Oberflächen mit Streptavidin beschichtet). Die Probe wird/kann (mehrere Male) über eine derartig funktionalisierte Oberfläche/Körper gespült und anschließend wieder entnommen/ entnommen werden. Ein großer Teil der zuvor nicht-gebundenen FISH-Sonden wird (kann) dabei auf dieser Oberfläche / diesem Körper immobilisiert und somit die aus den überschüssigen FISH-Sonden entstehen Hintergrundfluoreszenz aus dem Verfahren entfernt (werden).
- Fluoreszenzzerstörung überschüssiger FISH-Sonden vor Messung: Die Fluorophore der überschüssigen (nicht an die Ziel-RNA/DNA gebundenen) FISH-Sonden können physikalisch, chemisch oder biologisch so verändert werden, dass sie keine für die Messung relevante Fluoreszenz aufweisen. Dies kann z.B. durch die Zugabe von Reagenzien (z.B. Enzyme) erreicht werden (z.B. P450-Monooxygenasen), welche z.B. aromatische Strukturen der Fluorophoren modifizieren (z.B. hydroxylieren) und somit die relevanten Fluoreszenzeigenschaften verändern oder unterbinden. Derartige Reagenzien werden dabei erst nach dem Annealingschritt der FISH-Sonden hinzugeben. Die Methode wird dabei so gewählt, dass die Fluorophore der innerhalb der Zielorganismen gebundenen FISH-Sonden nicht betroffen sind, da sie z.B. durch die Zellmembran des Zielorganismus geschützt sind und so das Fluoreszenzinhibierende Reagenz nicht in deren Nähe gelangen bzw. nicht mit diesen interagieren kann.
- Reduktion des fluoreszierenden Hintergrundes durch den Einsatz von "freien" Quencher-Molekülen in Konzentrationen größer als 1mM. Die hohe Konzentration an freien Quencher-Molekülen bewirkt (kann) eine präferierte Löschung bzw. Reduktion der Fluoreszenz von freien/überschüssigen Oligosonden (z.B. linear, molekular beacon, scorpions etc.) außerhalb der markierten Mikroorganismen (bewirken). Die Löschung der intrazellulär gebundenen Fluoreszenz-Sonden durch freie Quencher-Moleküle wird (kann) durch die Tatsache vermindert (werden), dass die Diffusion der freien Quencher-Moleküle in die Zelle und die Verteilung innerhalb der Zelle durch die Zellbestandteile erschwert bzw. verhindert wird. Die Auswahl der Quencher-Moleküle hängt (kann) vom zu quenchenden Farbstoff ab(hängen). Für die Farbstoffe FAM, Alexa488, Atto488 u.Ä. eignen sich z.B. die Isomere von Methylrot (para-Methylrot, meta-Methylrot, o-Methylrot; 4- bzw.3- bzw. 2-{[4-(Dimethylamino)phenyl]diazenyl}benzoesäure).
- Immobilisierung der Zielorganismen: Die Zielorganismen können in einer besonders vorteilhaften Ausgestaltung des Messsystems (z.B. einem "Lab on a Chip"-System) auf Strukturen wie Filtern und insbesondere Track Etched Membranes zurückgehalten und/oder aufkonzentriert werden. Somit ist es möglich die Zielorganismen vom restlichen Probenansatz abzuscheiden und die Zielorganismen zusätzlich mit Spülsubstanzen von überschüssiger FISH-Sonden zu befreien, während markierte Zielorganismen zurückgehalten werden. Das hier beschriebene FISH-Verfahren kann dabei auch derartig ausgestaltet sein, dass die beschriebenen Reaktionsschritte, durch die sequentielle Zugabe aller benötigten Reagenzien, direkt auf der Filterstruktur (z.B. Track Etched Membran), durchgeführt werden. Hierdurch können auch die eingesetzten Reagenzien in Ihrer Stoffmenge reduziert und somit Herstellungskosten eingespart werden. Dazu ist (kann) es notwendig (sein) die Reagenzien einzeln auf die Filterstruktur zu spülen (z.B. durch pneumatischen und/oder zentrifugalen Transport). Besonders vorteilhaft sind Auslegungsformen, in denen die Zielorganismen zunächst auf eine Filterstruktur aufgespült und anschließend (innerhalb des mikrofluidischen Systems) wieder eluiert werden können, z.B. durch Eluieren/Abspülen mit einer Flüssigkeit entgegen der Aufspülrichtung / von der den Zielorganismen abgewandten Filterseite aus. Die Filterstruktur sollte dabei in ihren Eigenschaften (wie Eigenfluoreszenz) so ausgelegt sein, dass auch eine direkte Auslesung des Messergebnisses auf der Filterstruktur erfolgen kann.
- Metering innerhalb des fluidischen Systems: Das hier beschriebene FISH-Verfahren gestattet es breite Abweichungen eingebrachter Probenvolumina auszugleichen / abzupuffern. Hierzu ist es notwendig bestimmte Reagenzien und insbesondere die FISH-Sonden in hohem Überschuss zuzusetzen, um auch bei unerwartet hohen Probenvolumina über eine ausreichend hohe Konzentration von FISH-Sonden zur verfügen. Ein hoher Überschuss der FISH-Sonden zeichnet sich jedoch auch durch eine hohe Hintergrundfluoreszenz aus. Ziel sollte es daher sein, mit der minimalen und exakt abgestimmten Konzentration FISH-Sonden arbeiten zu können. Hierzu wird auf einer fluidischen Plattform zunächst ein Meteringschritt durchgeführt, welcher das Ausgangsprobenvolumen verlustfrei und per z.B. Zentrifugalkraft standardisiert ("metert"). Durch das nun bekannte Ausgangsvolumen können die FISH-Sonden und restlichen Reagenzien exakt in ihrer Konzentration eingestellt und somit innerhalb ihres Perfomance-Optimums verwendet werden. Hierzu wird zunächst die zu untersuchenden Probe z.B. per Zentrifugalkraft eingebracht und anschließend mit einem Überschuss einer Pufferflüssigkeit bis zu einem Überlaufkanal aufgefüllt. Nicht benötigte Pufferflüssigkeit wird über einen Überlaufkanal abgeführt. Die Position des Überlaufkanals ist dabei so gewählt, dass das Flüssigkeitsvolumen bis zum Überlaufen dieser "Meteringkammer" bekannt ist. Durch das Design der "Meteringkammer" ist dabei sichergestellt, dass kein Probenmaterial verlorengeht.

Das hier beschrieben FISH-Verfahren gestattet (kann) Aussagen über die Vitalität von untersuchenden Organismen anhand derer rRNA-Konzentration (gestatten). Ziel ist (kann) es (sein) die Unterscheidung zwischen "lebenden" und "toten" Mikroorganismen schnell und sehr spezifisch zu erreichen. Das hier beschrieben FISH-Verfahren basiert (vermutlich) generell auf dem Abbau von rRNA toter Mikroorganismen bzw. einem Aufbau von rRNA von lebenden Mikroorganismen zur Unterscheidung der Vitalität der Mikroorganismen. Die unterschiedliche Membran-Permeabilität von lebenden und toten Organismen kann jedoch ebenfalls genutzt werden, um lebende und tote Organismen schneller voneinander zu unterscheiden. Annahme dabei ist, das tote Zellen eine deutlich erhöhte Permeabilität der Zellmembran aufweisen. Mit den folgenden Optionen ist es möglich die Detektionsschwelle für "lebende" Mikroorganismen herabzusetzen, da toten Mikroorganismen nicht mehr (ausreichend) markiert werden und lebende Mikroorganismen (für deren Signalverstärkung bzw. Erhöhung des Unterschieds zwischen lebenden und toten Mikroorganismen) keine zusätzliche Nukleinsäuren (z.B. rRNA) für die Unterscheidung lebend/tot aufbauen müssen. Somit kann das Verfahren signifikant beschleunigt werden:
- Target Depletion: Die Ziel-Nukleinsäuren (wie rRNA) können vor dem eigentlichen Verfahren in toten Mikroorganismen abgebaut werden. Somit ist es möglich die Detektionsschwelle für "lebende" Mikroorganismen herabzusetzen und weniger rRNA-Aufbau (Zeit) dieser abzuwarten, da tote Mikroorganismen, durch die ihnen fehlenden Zielsequenzen nicht mehr oder kaum noch durch das Verfahren markiert werden können. Hierzu können entweder Ribonukleasen (wie Ribonuklease A) allein zugesetzt werden, oder Kombinationen aus Ribonukleasen (z.B. Ribonuklease H) mit Nukleinsäuren (z.B: DNA-Oligos). Im ersten Fall baut Ribonuklease A die ihr zugänglichen Gesamt-RNA ab. Im zweiten Fall binden die eingebrachten Nukleinsäuren spezifisch an die abzubauenden Nukleinsäuren (z.B. rRNA) und die Ribonuklease H erkennt den Heteroduplex aus eingebrachter DNA und der Ziel-rRNA. Dieser Heteroduplex wird nun durch die Ribonuklease H spezifisch abgebaut. Den Ribonukleasen und Nukleinsäuren ist es dabei nicht möglich die Membranen lebender Mikroorganismen zu durchdringen - somit werden lediglich Nukleinsäuren toter Organismen mit ausreichend permeablen Zellmembranen abgebaut. Hierzu können auch weitere Strukturen ("Ankerstrukturen") an z.B. die DNA-Oligos synthetisiert werden, welche die Durchdringung intakter Zellmembranen weiter erschweren. Generell ist bei diesem Vorgehen darauf zu achten, dass die eingebrachten Ribonukleasen vor Zugabe der FISH-Sonden entweder inaktiviert, gehemmt oder aus dem Probenansatz (z.B. durch Spülschritte auf einem Filter / track etched membran) entfernt werden. Zusätzlich kann diese Methode auch mit Detektionsverfahren kombiniert werden, welche z.B. auf dem Försterresonanzenergietransfer basieren oder das Auslesen mehrere Markierungen in einem Mikroorganismus ermöglichen. So können beispielsweise Proteine oder DNA als Fluoreszenzdonor (oder -Akzeptor) oder als zweite Zielstruktur für Markierungen verwendet werden und vor Beginn des eigentlichen Verfahrens bei toten Zellen abgebaut werden (z.B. durch Proteinasen oder Desoxyribonuklease, welche intakte Membranen lebender Organismen nicht durchdringen können). Somit werden lediglich lebende Mikroorganismen durch das Verfahren erfasst, da toten Zellen entweder über keinen Fluoreszenzdonor (oder -Akzeptor) und somit über keine z.B. Fluoreszenz im relevanten Emissionsbereich verfügen, oder lediglich über die Fluoreszenz der FISH-Sonden, nicht aber über die Fluoreszenz des Lebend/Tot-Indikators (z.B. nicht mehr vorhandene DNA oder Proteine) und somit lediglich einfach-markiert sind.
- Target Blocking: Die unterschiedliche Membran-Permeabilität von lebenden und toten Mikroorganismen kann genutzt werden, um Nukleinsäure (z.B. DNA-Oligos) in tote Mikroorganismen einzubringen und damit deren Untersuchungs-relevante Bindungsstellen (z.B. in deren rRNA) zu besetzen. Anschließend können z.B. FISH-Sonden nicht mehr an diesen Positionen binden, da die Stellen bereits besetzt sind. Es ist darauf zu achten, dass die eingebrachten Oligos zur Besetzung der relevanten Bindungsstellen, vor Einbringung der FISH-Sonden bzw. vor Permeabilisierung der lebenden Mikroorganismen, aus dem Probenansatz entfernt werden. Dies kann beispielsweise durch das Spülen des Probenansatzes über einen Filter (z.B. Track Etched Membran) erfolgen.

Einsatz von Lebend/Tot-Färbungen: Es ist möglich das hier verwendete FISH-Verfahren mit einer zusätzlichen lebend/tot-Unterscheidung der relevanten Mikroorganismen durchzuführen. Hierzu können die Zielorganismen auf einem Filter (wie einer track etched Membran) fixiert und mit einem Lebend/Tot-Farbstoff (wie z.B. Propidiumiodid) behandelt werden. Die Membran wird durch einen Sensor kartiert und für die jeweiligen Mikroorganismen der Zustand "lebend" oder "tot" festgehalten. Anschließend oder gleichzeitig wird das FISH-Verfahren durchgeführt und die durch die FISH-Sonden positivmarkierten Mikroorganismen zusätzlich mit dem Zustand "lebend-" oder "tot-" in der Datenerfassung versehen. Darüber hinaus können die Mikroorganismen auch mit dem hier beschriebenen FISH-Verfahren markiert und zusätzlich mit einem Lebend- oder Tot-Farbstoff (z.B. Propidiumiodid) versehen werden, falls dieser über andere spektrale Eigenschaften als die Farbstoffe der FISH-Sonden verfügt. Anschließend werden je Zielorganismus mehrere spektrale Eigenschaften (z.B. Fluoreszenzen in verschiedenen Wellenlängen bzw. Spektren) ausgelesen und Informationen zu z.B. Organismusart und deren Vitalität gleichzeitig aufgenommen.

Eine vorteilhafte Ausführungsform gemäß der Erfindung sieht vor, dass das Verfahren mit einem fluidischen Kanalsystem ausgeführt wird. Beispielsweise kann ein fluidisches Kanalsystem einen scheibenförmigen Probenträger umfassen. Von Vorteil ist dabei, dass ein spezifischer Nachweis von Mikroorganismen in unterschiedlichen Applikationsfeldern ermöglicht werden kann. Beispielsweise kann das erfindungsgemäße Verfahren zur mikrobiologischen Lebensmittelanalyse, Hygienekontrolle, klinischen und biotechnologischen Anwendungen sowie Umweltanalyse zum Einsatz kommen.

Eine bevorzugte Anwendung sieht ein fluidisches Kanalsystem vor, mit Mitteln zum Durchführen des Verfahrens, insbesondere wie zuvor beschrieben und/oder nach einem der auf ein Verfahren gerichteten Ansprüche. Beispielsweise können in dem fluidischen Kanalsystem zum Durchführen des erfindungsgemäßen Verfahrens ein Detektionsbereich und ein Vorbereitungsbereich ausgebildet sein. Insbesondere kann vorgesehen sein, dass die Querschnitte der Kanäle des fluidischen Kanalsystems auf Abmessungen der Mikroorganismen angepasst sein können.

Das fluidische Kanalsystem kann beispielsweise in Form eines Probenträgers ausgebildet sein. Der Probenträger kann insbesondere mindestens eine Kavität mit mindestens einer Nukleinsäuresonde und mindestens einer Substanz umfassen, die eine unterschiedliche Veränderung der Nukleinsäure-Konzentration, insbesondere RNA-Konzentration und/oder DNA-Konzentration, in toten Zellen und in lebenden Zellen begünstigt. Alternativ oder zusätzlich kann der Probenträger mit Mitteln zur optischen Zählung von markierten Mikroorganismen vorgesehen sein.

Der Probenträger kann als ein scheibenförmiger Probenträger ausgebildet sein. Beispielsweise kann der Probenträger als ein flacher Probenträger ausgebildet sein. Von Vorteil ist dabei, dass durch die Scheibenform des Probenträgers die Zentrifugalkraft für die Fluidförderung ausgenutzt werden kann. Die Fluidförderung ist auch über Druck oder auf eine andere Weise erreichbar. Alternativ kann der Probenträger eine dreidimensionale Erstreckung, beispielsweise in Form eines Zylinders oder küvettenartig, aufweisen.

Beispielsweise kann die Scheibenförmigkeit rotationssymmetrisch ausgebildet sein. Dies kann für ein Zentrifugieren von Vorteil sein. Es sind auch alternativ rechteckförmige Probenträger wie bei einer Chipkarte oder segmentförmige Probenträger wie bei einem Pizzastück bildbar.

Die Erfindung wird nun anhand von Ausführungsbeispielen näher beschrieben, ist aber nicht auf diese Ausführungsbeispiele beschränkt. Weitere Ausführungsbeispiele ergeben sich durch Kombination der Merkmale einzelner oder mehrerer Schutzansprüche untereinander und/oder mit einzelnen oder mehreren Merkmalen der Ausführungsbeispiele.

Es zeigt:
- Fig. 1: ein herkömmliches, Fluoreszenz-In-Situ-Hybridisierung (FISH)-basiertes Verfahren in einer schematischen Darstellung,
- Fig. 2: ein erfindungsgemäßes FISH-Verfahren in einer schematischen Darstellung,
- Fig. 3: das Verfahren gemäß Fig. 2 in einer detaillierten Darstellung.

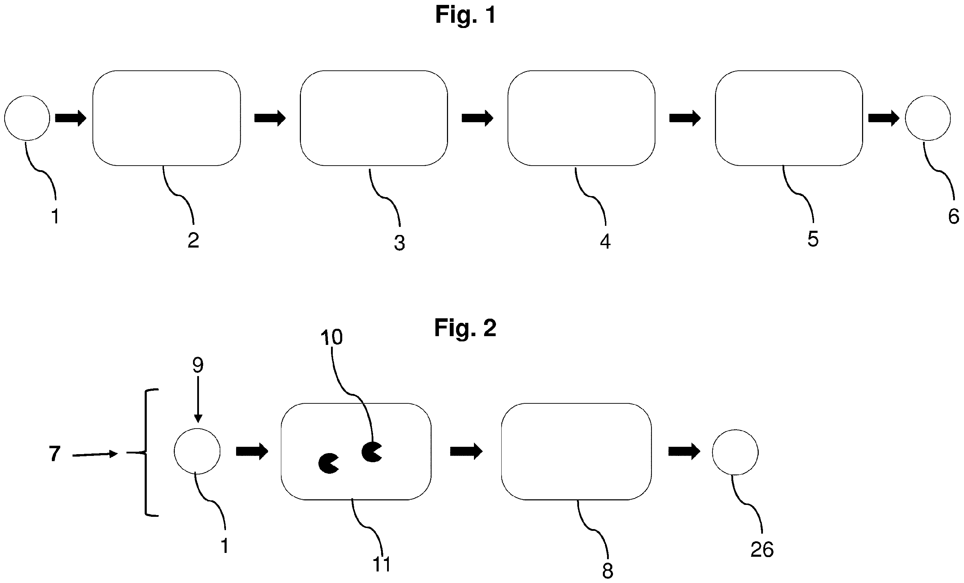

In den Figuren 1 bis 3 ist ein Verfahren zum Nachweis von Mikroorganismen in verschiedenen Ausführungen gezeigt.
- Fig. 1: zeigt ein herkömmliches FISH-Verfahren zum spezifischen Nachweis von Nukleinsäuren in einzelnen Mikroorganismen 1, umfassend die folgenden Schritte: Fixierung und Permeabilisierung 2 der in einer Probe enthaltenen Mikroorganismen 1; Waschen 3, um die zur Permeabilisierung verwendeten Reagenzien zu entfernen; Inkubation der fixierten und permeabilisierten Mikroorganismen 1 mit Nukleinsäuresonden, um eine Hybridisierung 4 herbeizuführen; Entfernen bzw. Abwaschen 5 der nicht hybridisierten Nukleinsäuresonden; und anschließende Analyse 6 der mit den Nukleinsäuresonden hybridisierten Mikroorganismen 1.
- Fig. 2: zeigt ein erfindungsgemäßes Verfahren 7 zum spezifischen Nachweis von Mikroorganismen 1, welches die Schritte der Permeabilisierung, Fixierung und Hybridisierung in einen einzigen Verfahrensschritt 8 zusammenfasst. Es werden keine zusätzlichen Waschschritte oder Pufferaustausche benötigt. Vor der Hybridisierung wird die Mikroorganismen-haltige Probe 9 beispielsweise mit Enzymen 10 inkubiert 11.

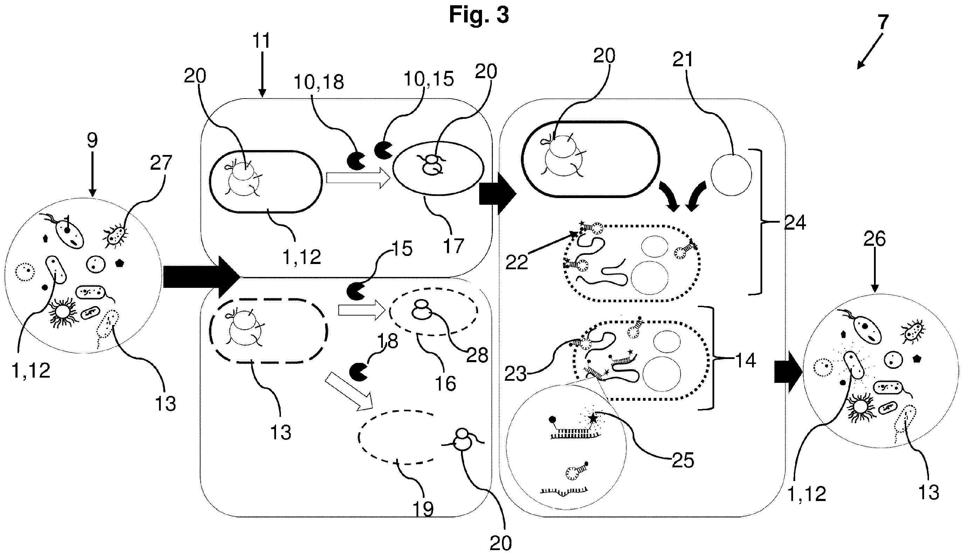

- Fig. 3: zeigt ein erfindungsgemäßes Verfahren 7 zum Nachweis von lebenden Mikroorganismen 1 und/oder zur Differenzierung von lebenden 12 und toten 13 Mikroorganismen in einer Probe 9 mittels in-situ-Hybridisierung 14, insbesondere einer Fluoreszenz-in-situ-Hybridisierung, und einer optischen Analyse 26 der nachzuweisenden Mikroorganismen 1, wobei der Probe 9 vor der Hybridisierung 14 wenigstens eine Substanz 15 beigegeben wird, die eine unterschiedliche Veränderung der Nukleinsäure-Konzentration, insbesondere RNA-Konzentration und/oder DNA-Konzentration, in toten Zellen 13 und in lebenden Zellen 12 begünstigt.

Durch die Inkubation 11 der Mikroorganismen 1 vor der Hybridisierung 14 mit Enzymen 10, die eine unterschiedliche Veränderung der RNA-Konzentration in toten Zellen und in lebenden Zellen begünstigt, können tote Partikel stark reduziert werden. Beispielsweise kann die rRNA des toten Mikroorganismus 13, der viel permeabler als ein lebender Mikroorganismus ist, durch die Einwirkung von RNAsen 15 enzymatisch abgebaut 16 werden, wobei gleicher Effekt bei den lebenden 12 Mikroorganismen ausbleibt 17. Der tote Mikroorganismus 13, bei dem der Schutz durch die äußere Membran als Diffusionsbarriere für große Moleküle, wie beispielsweise Enzyme, nicht mehr vorhanden ist, kann durch die Inkubation 11 mit Lysozymen 18 als Partikel komplett aufgelöst werden 19. Für Mikroorganismen mit einer stark ausgeprägten äußeren Hülle (Listerien, Staphylokokken etc.) sind (können) zusätzlich weitere lysierende Enzyme wie Mutanolysin, Staphylolysin etc. notwendig (sein). Die Ribosomen 20 bestehend aus RNAs und Proteinen können aus dem komplett oder teilweise aufgelösten Mikroorganismus diffundieren und können anschließend optisch nicht mehr nachgewiesen werden. Somit kann eine Differenzierung zwischen lebenden 12 und toten 13 Mikroorganismen und der Nachweis von ausschließlich lebenden Mikroorganismen ermöglicht werden.

Bei einer bevorzugten Anwendung wurde zum spezifischen Nachweis von lebenden Mikroorganismen eine Mikroorganismen-haltige Probe mit einem Hybridisierungspuffer (zum Beispiel aus einzelnen oder alle Bestandteilen aus der Liste: 900 mM NaCI, 20 mM Tris/HCl, 0,01 % SDS, 5,3 M Harnstoff, 1 mM EDTA, 0,13 µM Hybridisierungssonde und pH 8,0) versetzt und für einen Zeitraum von 15 bis 90 Minuten bei einer Temperatur von 52°C inkubiert. Für ListeriaFISH werden (können) 1250mM NaCI und kein Harnstoff in dem Hybridisierungspuffer benötigt (werden). Für Mikroorganismen mit einer stark ausgeprägten äußeren Hülle (Listerien, Staphylokokken etc.) sind (können) zusätzlich weitere lysierende Enzyme wie Mutanolysin, Staphylolysin etc. notwendig (sein). Nach Ende dieser Inkubationszeit wurden die fertig hybridisierten Proben durchflusszytometrisch oder Fluoreszenz-mikroskopisch analysiert.

Vor der Hybridisierung wird die Probe mit Lysozym und DNAse oder RNAse und/oder einer Protease in einer Konzentration von jeweils unter 5 mg/L in einem mit Mikroorganismen kombinierbaren Puffer (beispielsweise PBS-Puffer, Peptonwasser oder Tris-HCl-Puffer) inkubiert, um Leben/Tot-Differenzierung zu verbessern.

Erfindungsgemäß wird somit vorgeschlagen, ein Verfahren 7 zum Nachweis von lebenden Mikroorganismen 1 und/oder zur Differenzierung von lebenden 12 und toten 13 Mikroorganismen in einer Probe 9 mittels in-situ-Hybridisierung 14, insbesondere einer Fluoreszenz-in-situ-Hybridisierung, und einer optischen Analyse 26 der nachzuweisenden Mikroorganismen 1 bereitzustellen, wobei der Probe 9 vor der Hybridisierung 14 wenigstens eine Substanz 10 beigegeben wird, die eine unterschiedliche Veränderung der RNA-Konzentration in toten Zellen und in lebenden Zellen begünstigt.

### Bezugszeichenliste

- 1: nachzuweisende Mikroorganismen
- 2: Fixierung und Permeabilisierung gemäß herkömmlichem FISH-Verfahren
- 3: Waschen gemäß herkömmlichem FISH-Verfahren
- 4: Hybridisierung gemäß herkömmlichem FISH-Verfahren
- 5: Waschen gemäß herkömmlichem FISH-Verfahren
- 6: Analyse
- 7: erfindungsgemäßes Verfahren
- 8: Permeabilisierung, Fixierung und Hybridisierung, bevorzugt umfasst in einem Verfahrensschritt
- 9: Probe mit Mikroorganismen
- 10: Enzyme
- 11: Inkubation der Probe mit Enzymen
- 12: lebende Mikroorganismen
- 13: tote Mikroorganismen
- 14: Hybridisierung
- 15: RNAse
- 16: RNA-Abbau bei toten Mikroorganismen
- 17: kein Effekt von RNAsen in lebenden Mikroorganismen
- 18: Lysozym
- 19: Auflösung des toten Mikroorganismus als Partikel durch Inkubation mit Lysozymen
- 20: Ribosom bestehend aus RNAs und Proteinen
- 21: Detergens
- 22: Einschleusen spezifischer Nukleinsäuresonden
- 23: Nukleinsäuresonde
- 24: Fixierung der Mikroorganismen
- 25: Marker
- 26: optische Analyse
- 27: weitere Mikroorganismen, die nicht nachzuweisen sind
- 28: Ribosom mit abgebauten RNAs

## Patentansprüche

1. Verfahren (7) zum Nachweis von lebenden Mikroorganismen (1) und/oder zur Differenzierung von lebenden (12) und toten (13) Mikroorganismen in einer Probe (9) mittels in-situ-Hybridisierung (14), insbesondere einer Fluoreszenz-in-situ-Hybridisierung, und einer optischen Analyse (26) der nachzuweisenden Mikroorganismen (1), **dadurch gekennzeichnet, dass** der Probe (9) vor der Hybridisierung (14) wenigstens eine Substanz (10) beigegeben wird, die eine unterschiedliche Veränderung einer Nukleinsäure-Konzentration, insbesondere einer RNA-Konzentration und/oder einer DNA-Konzentration, in toten Zellen und in lebenden Zellen begünstigt.

2. Verfahren (7) nach Anspruch 1, **dadurch gekennzeichnet, dass** die beigegebene Substanz (10) wenigstens einen chemischen Stoff, der RNA und/oder DNA abbaut, insbesondere eine RNAse (15) und/oder DNAse, und/oder einen RNA-Abbau und/oder DNA-Abbau begünstigt, insbesondere Ethylendiamintetraacetat (EDTA), Diethylentriaminpentaessigsäure (DTPA), Triethylentetraminhexaessigsäure (TTHA), RNA-Helikase, Polymerase, Chaperon, siRNA, und/oder wenigstens einen chemischen Stoff, der die Durchlässigkeit bei toten Zellen erhöht, insbesondere Murein-Hydrolase, Lysozym, Mutanolysin, Glukosaminidase, Peptidase, Amidase, und/oder ein Nährmedium, das einen RNA-Aufbau in lebenden Zellen begünstigt, enthält.

3. Verfahren (7) nach einem der vorangehenden Ansprüche, **dadurch gekennzeichnet, dass** die Mikroorganismen (1) zumindest mit Beigabe der Substanzen (10) inkubiert (11) werden, insbesondere vor der Hybridisierung (14), und/oder wobei die Inkubationszeit so bemessen wird, dass noch keine Zellteilung stattfindet.

4. Verfahren (7) nach einem der vorangehenden Ansprüche, **dadurch gekennzeichnet, dass** die Hybridisierung (14) mindestens zwei, vorzugsweise alle, der Schritte einer Fixierung (24), einer Permeabilisierung und einer Denaturierung, vorzugsweise in einem Hybridisierungspuffer, umfasst, und/oder dass der oder ein Hybridisierungspuffer als Denaturierungssubstanz eine ungiftige Substanz, insbesondere Guanidiniumchlorid und/oder Harnstoff, und/oder Guanidiniumthiocyanat und/oder Formamid enthält.

5. Verfahren (7) nach einem der vorangehenden Ansprüche, **dadurch gekennzeichnet, dass** der Probe (9) ein Detergens (21) zugegeben wird, bevor die Hybridisierung (14), insbesondere ein Fixierungsschritt, abgeschlossen ist, und/oder dass vor der Hybridisierung (14) eine Lebend-Tot-Differenzierung der in der Probe (9) enthaltenden Mikroorganismen (1, 12, 13, 27) erfolgt.

6. Verfahren (7) nach einem der vorangehenden Ansprüche, **dadurch gekennzeichnet, dass** der Hybridisierungspuffer mindestens ein Denaturierungsmittel und eine RNAse-inhibierende Substanz enthält.

7. Verfahren (7) nach einem der vorangehenden Ansprüche, **dadurch gekennzeichnet, dass** die RNAse-inhibierende Substanz ausgewählt ist aus der Gruppe, bestehend aus Guanidiniumchlorid, Guanidiniumthiocyanat, Formamid, Dithiothreitol (DTT), Diethylpyrocarbonat (DEPC), Oligovinylsulfonsäure, Polyvinylsulfonsäure, Ethansulfonsäure, Heparin, Gluthation, natürlichen Nukleosiden und Nukleotiden.

8. Verfahren (7) nach einem der vorangehenden Ansprüche, **dadurch gekennzeichnet, dass** der Hybridisierungspuffer mindestens ein Salz, vorzugsweise Natriumchlorid, Kaliumchlorid oder Magnesiumchlorid, enthält.

9. Verfahren (7) nach einem der vorangehenden Ansprüche, **dadurch gekennzeichnet, dass** die optische Analyse (26) einen Schritt der Detektion, vorzugsweise des Quantifizierens und/oder einer Einzeldetektion der Mikroorganismen mit hybridisierten Nukleinsäuresonden (23) umfasst.

10. Verfahren (7) nach einem der vorangehenden Ansprüche, **dadurch gekennzeichnet, dass** die Nukleinsäuresonde (23) komplementär zu einer RNA eines nachzuweisenden Mikroorganismus (1) ist.

11. Verfahren (7) nach einem der vorangehenden Ansprüche, **dadurch gekennzeichnet, dass** die Nukleinsäuresonde (23) ausgewählt ist aus der Gruppe, bestehend aus linearen Oligonukleotidsonden, vorzugsweise monoProbes, dopeProbes, tetraProbes, multiProbes, und/oder Nukleinsäuresonden mit Sekundärstruktur, vorzugsweise Molecular Beacons und Scorpions Sonden.

12. Verfahren (7) nach einem der vorangehenden Ansprüche, **dadurch gekennzeichnet, dass** die Nukleinsäuresonde (23) mit einem detektierbaren Marker (25) verbunden ist und/oder der detektierbare Marker (25) ausgewählt ist aus der Gruppe, bestehend aus Fluoreszenzmarkern, Chemolumineszenzmarkern, Affinitätsmarkern und enzymatisch aktiven Gruppen.

13. Verfahren (7) nach einem der vorangehenden Ansprüche, **dadurch gekennzeichnet, dass** das Verfahren (7) mit einem fluidischen Kanalsystem ausgeführt wird.

14. Verfahren (7) nach einem der vorangehenden Ansprüche, **dadurch gekennzeichnet, dass** das Verfahren (7) einen Schritt zur zusätzlichen Hintergrundreduktion, vorzugsweise durch Benutzung eins zweiten Farbstoffs gemäß/analog zum Försterresonanzenergietransfer, und/oder das Einbringen von Quenching-Oligos nach des Annealingschritts der FISH-Sonden, und/oder Immobilisierung von freien FISH-Sonden vor Messung, und/oder physikalische, chemische oder biologische Veränderung von Fluorophoren überschüssiger FISH-Sonden, und/oder Einsatz von "freien" Quencher-Molekülen in Konzentrationen größer als 1mM, und/oder Immobilisierung der Zielorganismen auf Strukturen wie Filtern und insbesondere Track Etched Membranes, und/oder Zusatz bestimmte Reagenzien und insbesondere FISH-Sonden in hohem Überschuss (Metering), umfasst.

15. Verfahren (7) nach einem der vorangehenden Ansprüche, **dadurch gekennzeichnet, dass** das Verfahren (7) einen Schritt zur verbesserten Lebend/Tot-Unterscheidung, vorzugsweise durch Abbau der Ziel-Nukleinsäuren (wie rRNA) (Target Depletion) in toten Mikroorganismen vor dem eigentlichen Verfahren (7), und/oder Einbringen von Nukleinsäuren (z.B. DNA-Oligos) in tote Mikroorganismen um damit Untersuchungs-relevante Bindungsstellen (z.B. in deren rRNA) (Target Blocking) zu besetzen, und/oder Einsatz von Lebend/Tot-Färbungen, umfasst.

16. Fluidisches Kanalsystem, insbesondere scheibenförmiger Probenträger, mit Mitteln zum Durchführen des Verfahrens (7) nach einem der Ansprüche 1 bis 13, insbesondere umfassend mindestens eine Kavität mit einer Nukleinsäuresonde (23) und mindestens einer Substanz (10), die eine unterschiedliche Veränderung der Nukleinsäure-Konzentration, insbesondere RNA-Konzentration und/oder DNA-Konzentration, in toten Zellen (13) und in lebenden Zellen (12) begünstigt, und/oder mit Mitteln zur optischen Zählung von markierten Mikroorganismen (1).
